(19)

Europäisches
Patentamt
European
Patent Office
Office européen
des brevets

(11) **EP 4 631 450 A2**

(12) **EUROPEAN PATENT APPLICATION**

(43) Date of publication:
   **15.10.2025  Bulletin 2025/42**

(21) Application number: **25198567.7**

(22) Date of filing: **28.07.2022**

(51) International Patent Classification (IPC):
   ***A61B 18/00*** *(2006.01)*

(52) Cooperative Patent Classification (CPC):
   **A61B 18/1206; A61B 34/25;** A61B 18/1482;
   A61B 2017/00026; A61B 2017/00119;
   A61B 2017/00199; A61B 2018/00029;
   A61B 2018/00178; A61B 2018/00434;
   A61B 2018/00565; A61B 2018/00577;
   A61B 2018/00648; A61B 2018/00666;
   A61B 2018/00702; A61B 2018/00714;     (Cont.)

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**

(30) Priority:  **30.07.2021   US 202163227387 P**

(62) Document number(s) of the earlier application(s) in
   accordance with Art. 76 EPC:
   **22755030.8 / 4 376 743**

(71) Applicant: **Stryker Corporation
   Portage, MI 49002 (US)**

(72) Inventors:
   • **SPRINKLE, Thomas Bowen
     Kalamazoo, MI 49008 (US)**
   • **KIDMAN, Beau Michael
     Kalamazoo, MI 49009 (US)**
   • **HINES, Douglas Paul
     Kalamazoo, MI 49008 (US)**
   • **LATCHFORD, Blake William
     Vicksburg, MI 49097 (US)**

(74) Representative: **Röthinger, Rainer
   Wuesthoff & Wuesthoff
   Patentanwälte und Rechtsanwalt PartG mbB
   Schweigerstrasse 2
   81541 München (DE)**

Remarks:
   This application was filed on 28.08.2025 as a
   divisional application to the application mentioned
   under INID code 62.

(54)   **ELECTROSURGICAL CONSOLE FOR RADIOFREQUENCY ABLATION**

(57)   An electrosurgical system for radiofrequency ablation. An electrosurgical console includes a processor configured to determine impedance threshold values based on an initial tissue impedance value. The impedance threshold values may vary based on an elapsed time-based characteristic. A temperature set point is reduced based on the occurrence of the impedance event in which a tissue impedance value exceeds a threshold value. The reduced temperature set point may be fixed. The controller is configured to control the delivery of the RF energy drive the tissue temperature to the temperature set point(s). The temperature set point may not be reduced if the impedance event occurs during a ramp period, and an alert may be displayed. Secondary impedance threshold values may be utilized, and slower ramp periods may be provided for later in the ablation procedure. Methods of performing the ablation procedure with the electrosurgical system are also disclosed.

FIG. 1

**(Cont. next page)**

(52) Cooperative Patent Classification (CPC): (Cont.)
A61B 2018/00761; A61B 2018/00821;
A61B 2018/00875; A61B 2018/00886;
A61B 2018/00898; A61B 2018/167;
A61B 2034/254; A61B 2218/002; A61B 2560/0443

## Description

PRIORITY CLAIM

This application claims priority to and the benefit of United States Provisional Application No. 63/227,387, filed on July 30, 2021, which is hereby incorporated by reference in its entirety.

BACKGROUND

Radiofrequency (RF) energy is commonly utilized to ablate diseased tissue to treat pain or pathology. The tissue may be sensory nerves, intraosseous nerves, or intraosseous tumors, among other anatomic structures. Conventionally, an electrode is coupled to an electrosurgical console, and the RF energy is conducted from the electrode to the tissue across an electrode-tissue interface to create a lesion at the treatment location. For intraosseous tumors, the RF energy often heats the tissue to at least 90°C (194°F) to destroy the cells of the tumor.

The tissue has a tissue impedance, that is, its natural resistance to conducting the RF energy. It has been observed that as tissue becomes dehydrated or charred, the tissue impedance may increase rapidly. In other words, the charring may undesirably change the electrical and thermal conductivity of the tissue. Thus, detecting an increase of the tissue impedance may signal that the effectiveness of the RF energy being delivered to the treatment location is being compromised. Therefore, preventing or properly accounting for increases in the tissue impedance is an area of interest and development.

It has been shown that the infusion of a fluid, such as saline, to the treatment location may limit charring by improving conductivity across the electrode-tissue interface and by decreasing the tissue temperature. Known solutions have also included impedance-controlled systems in which the delivery of the RF energy is suspended when the tissue impedance increases beyond a predetermined threshold. The sudden suspension of RF energy is disruptive to the surgical procedure. Moreover, with the effects of charring being only partially reversible, efforts to return the tissue to the previous temperature set point often can result in further, repeated spikes in the tissue impedance. A fraught cycle may ensue in which there is significant disruption and downtime during the ablation procedure, and wherein achieving the desired lesion may become unfeasible.

SUMMARY

The present disclosure is directed to an electrosurgical system, electrosurgical console, and computer-implemented methods that overcome the aforementioned shortcomings. The electrosurgical system includes electrosurgical instrument(s) and, optionally, a cable accessory and/or a ground pad. The cable accessory is configured to be removably coupled with the electrosurgical console, and the electrosurgical instruments are configured to be removably coupled with the electrosurgical console and/or the cable accessory. An infusion module may be operable with the electrosurgical instrument to direct a fluid through the electrosurgical instrument. A thermocouple is positioned near the distal end of the electrosurgical instrument and configured to provide a temperature measurement of the tissue near the electrode(s). The infusion module includes an infusion clamp configured to be actuated to selectively start and stop the flow of the fluid. The electrosurgical console generates electrical energy of a controlled radiofrequency and passes the energy through the electrosurgical instrument and the tissue, thereby heating the tissue to sufficient temperature to destroy cells of the tissue. The electrosurgical console includes a display configured to display a graphical user interface (GUI) for enabling the user, among other actions, to select operating parameters and to navigate through different modes of operation provided by software on the electrosurgical console.

The electrosurgical console includes a controller, one or more processors, and memory. Computer-executable instructions or code may be stored on the memory, for example, within a database of the memory. The instructions are accessible by the processor, and executable by the processor to implement various functions of the electrosurgical console. The electrosurgical instrument is configured to be removably coupled with the electrosurgical console to arrange the electrosurgical instrument in communication with the controller. Based on input to the GUI, the controller controls the RF energy supplied to the electrosurgical instrument. The controller is further configured to receive or process one or more treatment parameters such as tissue temperature. Another treatment parameter includes tissue impedance, which may be determined by the processor based on measured electrical parameters, for example, supply and return voltages through the electrodes across the electrode-tissue interface. The processor may be configured to determine an initial tissue impedance value. The initial tissue impedance value may be based on initial supply and return voltages through the electrodes across the electrode-tissue interface.

The processor may be configured to determine impedance threshold values based on the initial tissue impedance value. The impedance threshold values that vary based on an elapsed time-based characteristic of the ablation procedure. The elapsed time-based characteristic may be an elapsed time from commencing delivery of the RF energy, or from another start or set time of the ablation procedure. The set time may be selected or predefined, for example, once the tissue temperature, as measured by the thermocouple, is near or at a temperature set point. The elapsed time may be continuous, or be paused during periods during which no RF energy is being delivered. The elapsed time-based characteristic may be a percentage of total ablation time.

The total ablation time may be inputted by the user to the GUI, or determined by the processor. The percentage of total ablation time may be a ratio of the elapsed time to the total ablation time. The elapsed time-based characteristic may be a percentage of the lesion being above a threshold temperature. The threshold temperature may be the temperature set point, a percentage of the temperature set point, or another determined temperature based on any number of factors of the ablation procedure. The processor may be configured to determine the percentage as a ratio of the elapsed time of the lesion being above the threshold temperature to the total ablation time. The processor is further configured to determine a final threshold impedance value. The final threshold impedance value may be the impedance threshold value for the last elapsed time-based characteristic, for example, at the end of the ablation procedure when the RF energy is terminated. This may correspond to one hundred percent of the lesion having been above the temperature threshold.

The impedance threshold values may be determined from a function, for example, according to an equation. The impedance threshold values may define an impedance threshold curve. The impedance threshold curve may be linear or non-linear and extend between the initial impedance value and the final impedance value. The impedance threshold curve may have a positive slope such that the threshold values being greater with higher values of the elapsed time-based characteristic. The slope of a respective one of the impedance threshold curves may decrease with higher values of the initial tissue impedance value. The impedance threshold curves may have different slopes such that an offset of the impedance threshold curves is greater at lower values of the elapsed time-based characteristic than at the higher values of the elapsed time-based characteristic. A subsequent one of the impedance threshold values may be based on the occurrence of an impedance event such that the impedance threshold values may deviate from a predefined function or curve. The impedance threshold values may be predefined at respective times of the ablation procedure.

The controller is configured to control the RF energy delivered from the electrode based on the temperature measurement of the thermocouple and the temperature set point at which the tissue is to be ablated. The processor is configured to reduce the temperature set point based on an occurrence of an impedance event. The electrosurgical console utilizes impedance-based control to drive the tissue temperature towards the temperature set point. The temperature set point may be predefined, determined, or user selected. The predefined temperature set point may be based on, for example, the type of lesion to be ablated. The determined temperature set point may be based on, for example, the type of lesion, the total ablation time, tissue impedance, or another treatment parameter. The processor may be configured to determine the temperature set point. The user-selected temperature set point may be inputted to the GUI by the user.

The RF energy is delivered from the electrode to drive the tissue temperature approximately to or exactly to the temperature set point. During the delivery of the RF energy, the processor is configured to determine subsequent tissue impedance values during delivery of the RF energy with the electrosurgical instrument. The processor compares the subsequent tissue impedance values with the impedance threshold values. Should the determined tissue impedance value not exceed the impedance threshold value at a given elapsed time-based characteristic of the ablation procedure, the processor determines that no impedance event has occurred. Should the processor determine an impedance event has occurred, the temperature set point is reduced. The above actions may be performed continuously and in real-time throughout the ablation procedure. The reduction of the temperature set point may be permanent. In other words, the temperature set point may not be increased for the remainder of the ablation procedure.

The reduction of the temperature set point may be a temperature offset. The temperature offset may be fixed or variable. The fixed temperature offset may be a value by which the temperature set point is reduced with each occurrence of an impedance event. The temperature offset may be fixed or varied, and/or predefined, determined, or user selected, and combinations thereof. The processor may be configured to determine the temperature offset based on the initial tissue impedance, the initial temperature set point, elapsed time between impedance events, the type of lesion, or any other treatment parameters. The determined temperature offset may be fixed, or iteratively determined after each occurrence of an impedance event. The user-selected temperature offset be inputted to the GUI by the user.

The ablation procedure includes a ramp period during which the tissue temperature is increasing towards the temperature set point. The electrosurgical console limits instances in which the temperature set point is reduced to the impedance events occurring after the ramp period. The processor is configured to determine whether the occurrence of the impedance event is during or after the ramp period. The processor may compare the tissue temperature to the temperature set point or another temperature based on the temperature set point. The temperature may be the threshold temperature in relation to the elapsed time-based characteristic. The threshold temperature may be a percentage of the temperature set point. The processor is configured to maintain the temperature set point if the occurrence of the impedance event is during the ramp period. The processor is further configured to reduce the temperature set point if the occurrence of the impedance event is after the ramp period. In instances where the occurrence of the impedance event is during the ramp period, the processor is further configured to instruct the display to provide an alert. The display may also display the total ablation time, operating mode, a graphical representation of a temperature versus time for the ablation procedure, an indication of the electrosurgical instrument coupled to the electrosurgical console, among other treatment parameters.

The processor may generate a secondary impedance threshold values to be utilized on conjunction with the impedance threshold values. The secondary impedance threshold values may be greater than those of the primary impedance threshold values for all elapsed time-based characteristics. The secondary impedance threshold values may be utilized in instances where the reduction in the temperature set point is insufficient to prevent a continued increase in tissue impedance. The secondary impedance threshold values may have the same or different slope as the primary impedance threshold values. Should the tissue impedance exceed the impedance threshold values of the secondary impedance threshold values, the processor may instruct the controller to suspend the delivery of the RF energy to the electrosurgical instrument. The pause of the delivery of RF energy provides time for the fluid from the infusion module to partially reverse the effect of the charring of the tissue or the like. After a predefined or determined period of time, the controller is configured to resume the delivery of the RF energy to the electrosurgical instrument.

The processor is configured to provide for an increase in a duration of ramp periods following occurrences of impedance events. Following a temporary suspension of the delivery of the RF energy in response to an impedance event, the speed at the tissue temperature is driven to the temperature set point is decreased. The tissue may be heated more slowly following restarts at later points in the ablation procedure, as a slower ramp period provides for a more homogeneous ablation volume.

BRIEF DESCRIPTION OF THE DRAWINGS

FIG. 1 is a perspective view of an electrosurgical system including an electrosurgical console, a cable accessory, electrosurgical instruments, an infusion module coupled to one of the electrosurgical instruments, and a ground pad assembly.

FIG. 2 is a graphical representation of impedance threshold trajectories in which impedance values are increasing as a function of a percentage of procedure time the tissue is above a predetermined temperature threshold.

FIG. 3 is a graphical representation of impedance as a function of percent of lesion above a temperature threshold with a plurality of exemplary impedance threshold curves illustrated.

FIG. 4 is a graphical representation of tissue temperature (in degrees Celsius) as a function of elapsed time of energy (in minutes). The instances of a decrease in the temperature set point are associated with occurrences of impedance events.

FIG. 5 is view of a display of the electrosurgical console showing operating parameters of the ablation procedure.

FIG. 6 is another view of the display in which an alert is shown in response to an impedance event occurring during a ramp period of the ablation procedure.

FIG. 7 is a graphical representation of tissue temperature (in degrees Celsius) as a function of elapsed time of energy (in minutes). A duration of ramp periods is shown as increasing at later points the ablation procedure.

FIG. 8 is a graphical representation of tissue temperature (in degrees Celsius) as a function of elapsed time of energy delivery (in minutes). The suboptimal arrangement does not provide for decreasing the temperature set point after occurrences of impedance events, and the charring of the tissue over time prevents the tissue from returning to near the temperature set point.

DETAILED DESCRIPTION

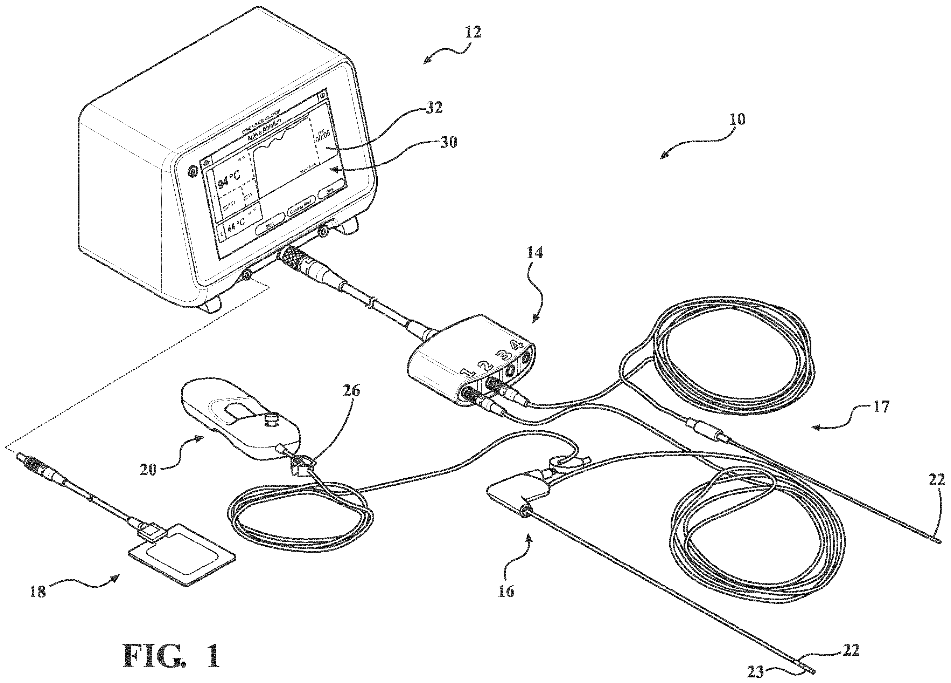

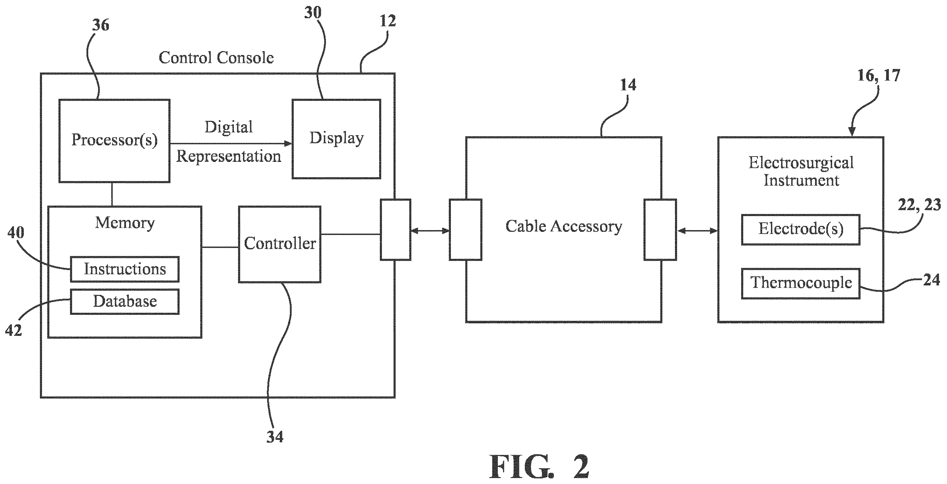

Referring to FIGS. 1 and 2, an electrosurgical system 10 includes an electrosurgical console 12, electrosurgical instrument(s) 16, 17 and, optionally, a cable accessory 14 and/or a ground pad 18. The cable accessory 14 is configured to be removably coupled with the electrosurgical console 12, and the electrosurgical instruments 16, 17 are configured to be removably coupled with the electrosurgical console 12 and/or the cable accessory 14. One implementation of the electrosurgical instrument 17 may be a monopolar electrode in which an electrode 22 is operable with the ground pad 18 removably couplable to the electrosurgical console 12. Another implementation of the electrosurgical instrument 16 is a bipolar electrode, for example, a self-grounding bipolar electrode in which proximal and distal electrodes 22, 23 provide a path of electrical current through the tissue to be ablated. More than one bipolar electrode may be used simultaneously, for example, in a bipedicular approach through the vertebral body. An infusion module 20 may be operable with the electrosurgical instrument 16 to direct a fluid through the electrosurgical instrument 16 to be discharged near proximal and distal electrodes 22, 23. A thermocouple 24 is positioned near the distal end of the electrosurgical instrument 16, 17 and configured to provide a temperature measurement of the tissue near the electrode(s) 22, 23. One suitable implementation of the electrosurgical instrument 16 and the infusion module 20 is disclosed in commonly-owned International Publication No. WO 2020/198150, published November 5, 2020, the entire contents of which are hereby incorporated by reference. The infusion module 20 includes an infusion clamp 26 configured to be actuated to selectively start and stop the flow of the fluid.

The electrosurgical system 10 is configured to treat the tissue, namely by radiofrequency (RF) ablation. The electrosurgical console 12 generates electrical energy of a controlled radiofrequency and passes the energy through the electrosurgical instrument 16, 17 and the tissue, thereby heating the tissue to sufficient temperature to destroy cells of the tissue. The ablation may be carried out through a monopolar configuration, a parallel bipolar configuration, or a self-grounding bipolar configuration as detailed in commonly-owned International Publication No. WO 2018/200254, published November 1, 2018, the entire contents of which are hereby incorporated by reference. In certain implementations, the electrosurgical system 10 is utilized to ablate nerves for pain management. In other implementations, the electrosurgical system 10 is utilized to ablate lesions, and in particular intraosseous tumors. An exemplary procedure of particular interest is ablation of tumors within the vertebral body. An introducer assembly is deployed through one or both pedicles to facilitate access to within the vertebral body, and the electrosurgical console 12 applies temperature-controlled, RF energy into the tumor. Nerves associated within or surrounding the tumor may also be ablated to provide pain relief. It should be appreciated that the electrosurgical system 10 of the present disclosure may be utilized to treat intraosseous tumors of long bones, the skull, mandible, ileum, and the like.

The electrosurgical console 12, as shown in FIG. 1, comprises a display 30 configured to display a graphical user interface (GUI) 32 for enabling the user, among other actions, to select operating parameters and to navigate through different modes of operation provided by software on the electrosurgical console 12. The display 30, in one example, is a touch screen, enabling selection of digital indicia represented on the display 30 using the location of a touching that may be capacitively sensed on the touch screen. Alternatively, the display 30 may be paired with a separate touch screen device, or may be controlled by peripheral input devices connected to the electrosurgical console 12, such as a mouse and/or a keyboard.

Referring to FIG. 2, the electrosurgical console 12 includes a controller 34, one or more processors 36, and memory 38. Computer-executable instructions 40 or code may be stored on the memory 38, for example, within a database 42 of the memory 38. The instructions 40 are accessible by the processor 36, and executable by the processor 36 to implement various functions of the electrosurgical console 12. For example, the instructions 40 are configured to implement the GUI 32 on the display 30. The other functions implemented by execution of the instructions 40 are described below. The processor(s) 36 and memory 38 may have any suitable configuration and may be of any suitable type to enable implementation of the functions described herein. Moreover, for convention, the present disclosure describes the processor 36 executing the instructions 40, and the controller 34 controlling the output from or actions of the electrosurgical console 12, such as controlling the RF energy supplied to the electrosurgical instrument 16, 17. It is understood that the controller 34 may be implemented by the processor 36, or may be a device separate from the processor 36. The processor 36 is in communication with the controller 34, or configured to be arranged in wired or wireless communication with the controller 34 where the controller 34 and the processor 36 are contained on separate devices. Any of the functions described herein may be implemented by the controller 34, the processor(s) 36, or a combination thereof.

The electrosurgical instrument 16, 17 is configured to be removably coupled with the electrosurgical console 12 to arrange the electrosurgical instrument 16, 17 in communication with the controller 34. Based on input to the GUI 32, the controller 34 controls the RF energy supplied to the electrosurgical instrument 16, 17. The controller 34 is further configured to receive or process one or more treatment parameters such as temperature of the tissue. In particular, the controller 34 is in communication with the thermocouple 24 of the electrosurgical instrument 16, 17 and configured to receive a signal indicative of tissue temperature at the treatment site. Another treatment parameter includes tissue impedance, which may be determined by the processor 36 based on measured electrical parameters, for example, supply and return voltages through the electrodes 22, 23 across the electrode-tissue interface. The tissue temperature may also be used to determine the impedance values.

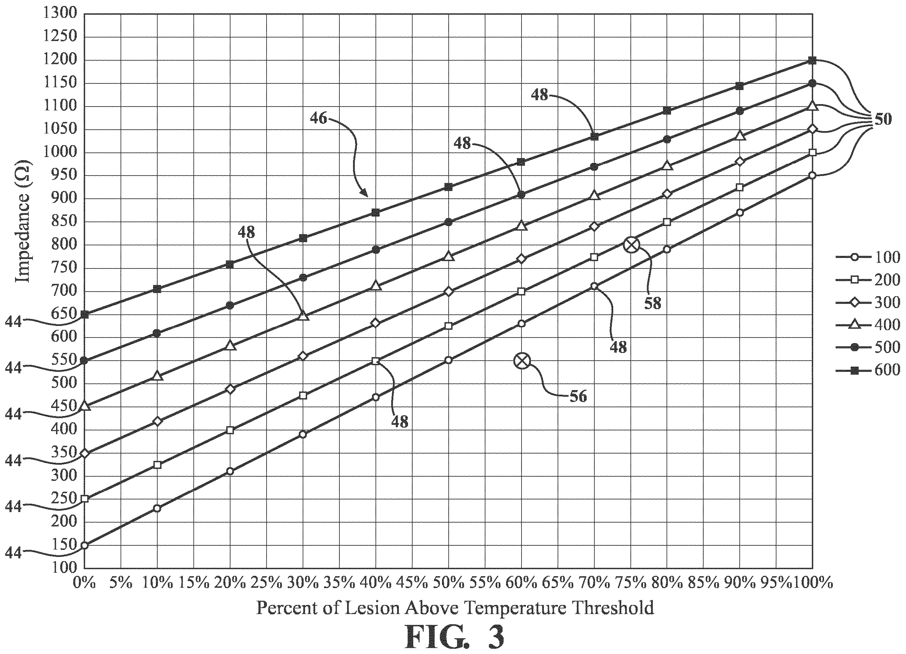

It is known that the tissue impedance changes while the lesion is being ablated, and higher tissue impedance values are more tolerable later in the ablation procedure. In other words, a decrease in the electrical and thermal conductivity of the tissue is of increasingly less concern after the lesion has been already heated for a longer period, whereas such a decrease earlier in the procedure may compromise the efficiency of the procedure. Referring now to FIG. 3, the processor 36 may be configured to determine an initial tissue impedance value 44. The initial tissue impedance value 44 may be based on initial supply and return voltages through the electrodes 22, 23 across the electrode-tissue interface. As such, the electrosurgical instrument 16, 17 may be deployed at the target site with the electrodes 22, 23 near, at, or within the lesion, and the path of electrical current is created for the initial supply and return voltages to be obtained. In one implementation, this may automatically occur once an input is provided to the GUI 32 to commence ablation. In other words, the initial tissue impedance value 44 may be determined in real-time with the delivery of the RF energy. Alternatively, after the electrosurgical instrument 16, 17 is deployed at the target site yet prior to commencing ablation, the user may select on the GUI 32 a mode to obtain tissue impedance, after which the path of electrical current of a subtherapeutic or sub-ablative magnitude is created for the initial supply and return voltages to be obtained, from which the initial tissue impedance value 44 is determined.

The processor 36 may be configured to determine impedance threshold values 48 based on the initial tissue impedance value 44. The impedance threshold values 48 may vary based on an elapsed time-based characteristic of the ablation procedure, reflective that higher tissue impedance values are more tolerable later in the ablation procedure. In a broadest sense, the elapsed time-based characteristic may be an elapsed time from commencing delivery of the RF energy, or from another start or set time of the ablation procedure. The set time may be selected or predefined, for example, once the tissue temperature, as measured by the thermocouple 24, is near or at a temperature set point to be described. The elapsed time may be continuous, or be paused during periods during which no RF energy is being delivered. In another implementation, the elapsed time-based characteristic may be a percentage of total ablation time. The total ablation time may be inputted by the user to the GUI 32, or determined by the processor 36. For example, based on a size of the lesion to be ablated and the temperature set point, the processor 36 is configured to determine the total ablation time. The percentage of total ablation time may be a ratio of the elapsed time, as previously described, to the total ablation time. In still another implementation, the elapsed time-based characteristic may be a percentage of the lesion being above a threshold temperature, as shown in FIG. 3. The threshold temperature may be the temperature set point, a percentage of the temperature set point, or another determined temperature based on any number of factors of the ablation procedure. The tissue temperature of the lesion is sensed by the thermocouple 24 and transmitted to the processor 36. The processor 36 may be configured to determine the percentage as a ratio of the elapsed time of the lesion being above the threshold temperature to the total ablation time, as previously described. In such an implementation, the percentage may increase despite RF energy being paused, provided the temperature remains above the temperature threshold. It has been shown empirically that using the percentage of the lesion being above the threshold temperature as a basis for the impedance threshold values more effectively provides for a desired ablation profile while accounting for increases and/or transient spikes in the tissue impedance.

In certain implementations, the impedance threshold values 48 may define an impedance threshold curve 46. The impedance threshold values 48 may be determined according to a function, for example, the equation:

$$Z_t(t) = Z_s + (Z_m - Z_s) * t_e,$$

where $Z_t(t)$ is the impedance threshold value 48 at a given time in the procedure, $Z_s$ is the initial impedance value 44, $Z_m$ is the maximum impedance threshold, and $t_e$ is the elapsed time-based characteristic, for example, a percentage of the total procedure time. Additional consideration may be given to a noise margin. The maximum impedance threshold and the noise margin may be fixed or variable values. The total procedure time may be variably determined prior to the procedure, or based on predefined defaults stored in the memory 38. The predefined defaults may be based on a size of the probe of the electrosurgical instrument 16, 17.

The processor 36 is further configured to determine a final threshold impedance value 50. The final threshold impedance value 50 may be the impedance threshold value 48 for the last elapsed time-based characteristic, for example, at the end of the ablation procedure when the RF energy is terminated. This may correspond to the elapsed time-based characteristic in the above equation being equal to one, and/or one hundred percent of the lesion having been above the temperature threshold. The final impedance threshold value 50 may be based on the initial tissue impedance value 44, and determined according to the equation. In alternative implementations, the memory 38 may store a plurality of impedance threshold curves, and the processor 36 is configured to determine the impedance threshold curve by selecting one of the impedance threshold curves 46 stored on the memory 38. One example includes comparing the initial tissue impedance value 44 with respective initial tissue impedance values of the impedance threshold curves 46, and selecting the impedance threshold curve 46 to which the initial tissue impedance value 44 is closest. Additionally or alternatively, a subsequent one of the impedance threshold values 48 may be based on the occurrence and/or the characteristics of an impedance event such that the impedance threshold values may be determined in real-time and deviate from a predefined function. One example includes the subsequent one of the impedance threshold values 48 being higher or lower based on the magnitude by which the tissue impedance value 44 exceeds the impedance threshold value 48.

FIG. 3 shows several exemplary impedance threshold curves 46 for illustrative purposes. The impedance threshold curve 46 may be linear and extend between the initial tissue impedance value 44 and the final impedance threshold value 50. The impedance threshold curve 46 may have a positive slope such that the threshold values being greater with higher values of the elapsed time-based characteristic. Further, the slope of a respective one of the impedance threshold curves 46 may decrease with higher values of the initial tissue impedance value 44. For example, and as shown in FIG. 3, the impedance threshold curves 46 have different slopes such that an offset of the impedance threshold curves 46 is greater at lower values of the elapsed time-based characteristic than at the higher values of the elapsed time-based characteristic. More specifically, the initial tissue impedance values 44 are shown as offset from one another by 100 Ohms, whereas the final threshold impedance value 50 are shown as offset from one another by 50 Ohms. Other characteristics of the impedance threshold curve 46 are contemplated, such as those that are non-linear by having hyperbolic and logarithmic shapes, and those that are stepwise, among others.

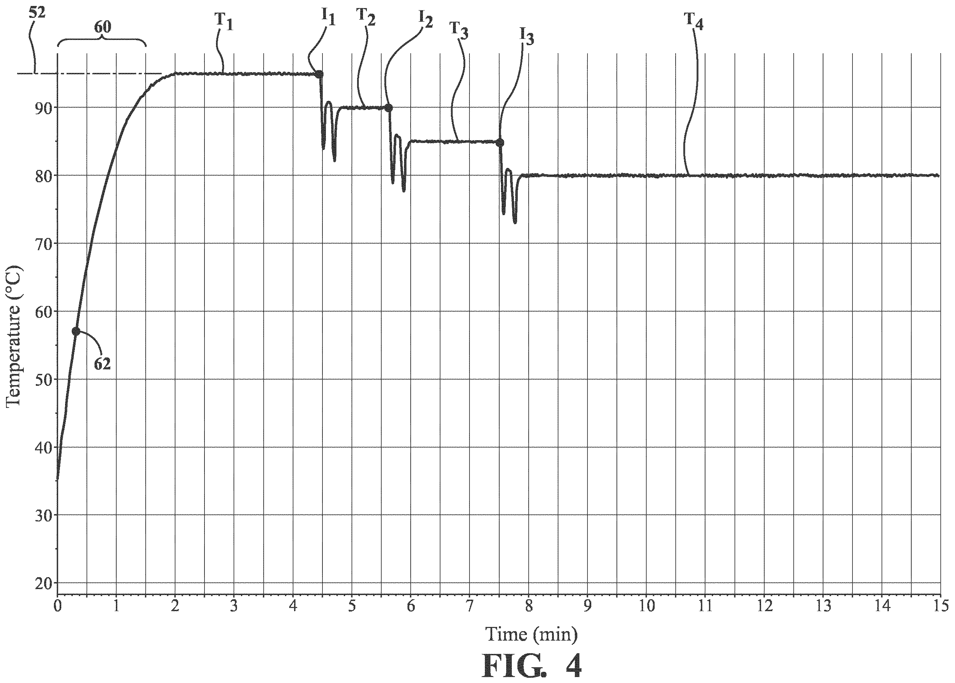

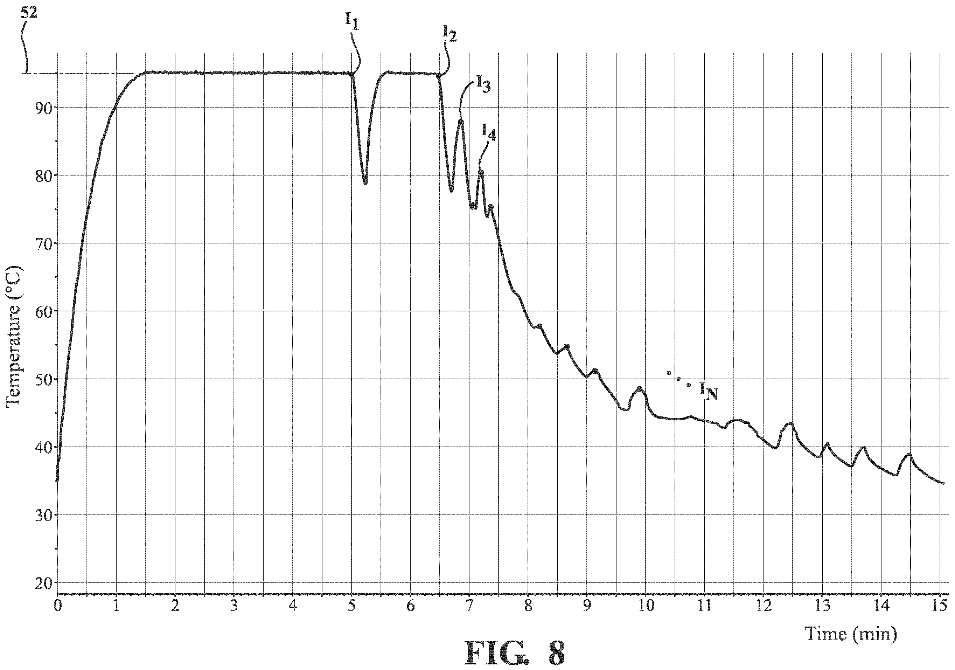

With further reference to FIG. 4, the processor 36 is configured to control the RF energy delivered from the electrode 22, 23 based on the temperature measurement of the thermocouple 24 and the temperature set point at which the tissue is to be ablated. Further, and as to be described, the processor 36 is configured to reduce the temperature set point based on an occurrence of an impedance event, *i.e.,* when the tissue impedance exceeds the impedance threshold value 48. The electrosurgical console 12 of the present disclosure, in which impedance-based control is utilized to drive the tissue temperature towards the temperature set point, advantageously provides for improved delivery of the RF energy with lessened risk of repeated, uncontrollable spikes in impedance. By contrast, known systems in which impedance is driven to an impedance trajectory may result in suboptimal temperature control. Similarly, FIG. 8 is reflective of a suboptimal arrangement in which the tissue temperature is driven towards a temperature set point; however, without reduction in the temperature set point following impedance events, repeated impedance events result in degradation of the electrode-tissue interface such that the deliverable RF energy insufficient for ablation. The electrosurgical console 12 of the present disclosure overcomes such shortcomings.

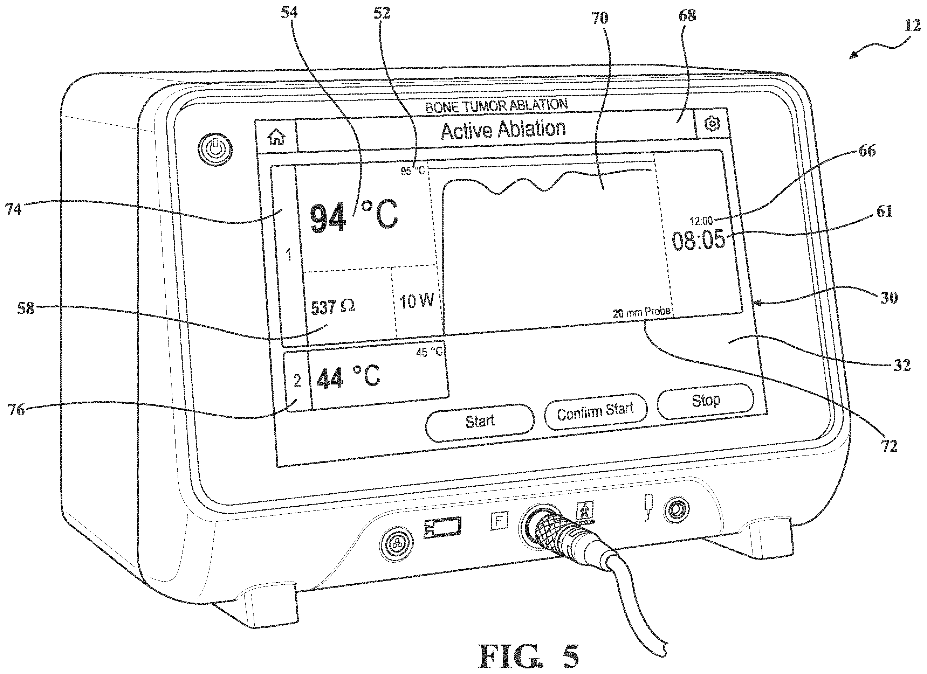

The temperature set point may be predefined, determined, or user selected. The predefined temperature set point may be based on, for example, the type of lesion to be ablated. Intraosseous tumors may be ablated at a temperature set point within the range of 90 to 100 degrees Celsius, more particularly within the range of 94 to 96 degrees Celsius, or another temperature indicated to destroy the cells of the tissue of the lesion. The determined temperature set point may be based on, for example, the type of lesion, the total ablation time, tissue impedance, or another treatment parameter. The processor 36 may be configured to determine the temperature set point. The user-selected temperature set point may be inputted to the GUI 32 by the user. FIG. 5 shows the temperature set point 52 being displayed on the display 30 as 95 degrees Celsius.

The RF energy is delivered from the electrode 22, 23 to drive the tissue temperature 54 approximately to or exactly to the temperature set point 52. FIG. 5 shows the tissue temperature 54 being displayed on the display 30 as 94°C. During the delivery of the RF energy, the processor 36 is configured to determine subsequent tissue impedance values during delivery of the RF energy with the electrosurgical instrument 16, 17. The processor 36 compares the subsequent tissue impedance values with the impedance threshold values 48. Should the determined tissue impedance value not exceed the impedance threshold value 48 at a given elapsed time-based characteristic of the ablation procedure, the processor 36 determines that no impedance event has occurred. As an example and with reference to FIG. 3, using the impedance threshold curve 46 with an initial tissue impedance value 44 or 150 Ohms, and for the elapsed time-based characteristic of 60%, a first tissue impedance value, represented at 56, is less than the impedance threshold value 48 of approximately 625 Ohms. The processor 36 may take no action and the controller 34 continues to deliver the RF energy to the electrode 22, 23 to drive the tissue temperature 54 towards the temperature set point 52. For the elapsed time-based characteristic of 75%, a second tissue impedance value, represented at 58, is greater than the impedance threshold value 48 of approximately 750 Ohms. The processor 36 determines the occurrence of an impedance event, and reduces the temperature set point 52. The above actions may be performed continuously and in real-time throughout the ablation procedure.

The reduction of the temperature set point 52 may be permanent. In other words, the temperature set point 52 may not be increased for the remainder of the ablation procedure. The underlying cause for the occurrence of the impedance event - for example, tissue charring or suboptimal contact between the electrode 22, 23 and the tissue - may remain, and efforts to return the temperature set point 52 to the initial temperature set point may result in additional occurrences of impedance events, as previously discussed. More particularly, FIG. 8 illustrates a first impedance event ($I_1$), after which the suboptimal arrangement attempts to drive the tissue temperature to the initial temperature set point. This may initially be feasible, perhaps due to the partial reversibility of the tissue charring following the temporary drop in tissue temperature, or due to improving contact between the electrode and the tissue. Yet, the characteristics of the lesion and/or the electrode-tissue interface may be unable to sustain the initial temperature set point, after which a second impedance event ($I_2$) occurs. The suboptimal arrangement again attempts to drive the tissue temperature to the initial temperature set point. Due to irreversible degradation of the electrode-tissue interface, third, fourth, and subsequent impedance events ($I_3$, $I_4$, $\cdots I_N$) occur at increasingly lower tissue temperatures. In other words, not only is the initial temperature set point unachievable but even more modest tissue temperatures result in impedance events. As a result, the tissue temperature at which RF energy may be delivered without an impedance event is insufficient for ablation whatsoever, thereby effectively rendering a failed ablation procedure.

Referring now to FIG. 4, an implementation of the present disclosure is illustrated in which the temperature set point 52 is reduced in response to the occurrence(s) of impedance events. With commencing delivery of the RF energy, the tissue temperature is ramped to the initial or first temperature set point ($T_1$). In the present example, the tissue temperature achieves the first temperature set point, and a first impedance event ($I_1$) occurs

after several minutes. The processor 36 reduces the first temperature set point ($T_1$) to a second temperature set point ($T_2$), and the controller 34 controls the RF energy to drive the tissue temperature to the second temperature set point ($T_2$). By doing so, the lesion and/or the electrode-tissue interface may be more likely to accommodate the level of RF energy associated with the second temperature set point, preferably without another occurrence of an impedance event. For illustrative purposes, FIG. 4 shows the occurrence of a second impedance event ($I_2$) and a third impedance event ($I_3$). Based on the occurrence of a second impedance event ($I_2$), the processor 36 reduces the second temperature set point ($T_2$) to a third temperature set point ($T_3$), and the controller 34 controls the RF energy to drive the tissue temperature to the third temperature set point ($T_3$). Likewise, based on the occurrence of a third impedance event ($I_3$), the processor 36 reduces the third temperature set point ($T_3$) to a fourth temperature set point ($T_4$), and the controller 34 controls the RF energy to drive the tissue temperature to the fourth temperature set point ($T_4$). The remainder of the ablation procedure proceeds with the lesion being ablated at the fourth temperature set point ($T_4$). In contrast to the suboptimal implementation of FIG. 8 where the tissue temperature decayed to approximately 40°C due to repeated occurrences of impedance events, the implementation of the present disclosure of FIG. 8 retains performance at 80°C, a temperature satisfactory to ablate the lesion. In other words, this approach continues to output RF energy and grow the ablation zone, albeit at a slower rate, and provides the user with confidence to proceed with the ablation procedure at set point temperature known to result in cellular destruction as intended.

The reduction of the temperature set point 52 is a temperature offset. The temperature offset may be fixed or variable. The fixed temperature offset may be a value by which the temperature set point is reduced with each occurrence of an impedance event. The fixed temperature offset may be within the range of 3 to 7°C, and more particularly approximately 5°C. The range of 3 to 7°C has been shown empirically to sufficiently reduce the likelihood of subsequent occurrences of impedance events. In the illustrative implementation of FIG. 4, the fixed temperature offset is 5°C such that the initial and first temperature set point is 95°C, the second temperature set point is 90°C, the third temperature set point is 85°C, and the fourth temperature set point 80°C, and so on. It is further contemplated that the temperature offset may be fixed or varied, and/or predefined, determined, or user selected, and combinations thereof. For example, the processor 36 may be configured to determine the temperature offset based on the initial tissue impedance, the initial temperature set point, elapsed time between impedance events, the type of lesion, or any other treatment parameters. The determined temperature offset may be fixed, or iteratively determined after each occurrence of an impedance event. In other words, a first temperature offset may be 3°C, but should a subsequent impedance event follow shortly thereafter, *e.g.,* within a predetermined or determined elapsed time, the second temperature offset may be five, seven, or more degrees Celsius. Alternatively, the user-selected temperature offset be inputted to the GUI 32 by the user. For example, the user-selected temperature offset may be selected as fifteen degrees Celsius such that the second temperature set point is 80°C as such a temperature has empirically demonstrated very little impedance rise in testing.

With continued reference to FIG. 4, the ablation procedure includes a ramp period 60 during which the tissue temperature is increasing towards the temperature set point 52. With the human body having a nominal temperature of 37°C, the ramp period 60 may be the first one, two, three, or four or more minutes of the ablation procedure before achieving the temperature set point 52 of, for example, 95°C. During the ramp period 60 where the tissue temperature is only forty, fifty, and sixty degrees Celsius, and the like, an occurrence of an impedance event may not be due to a decrease in the electrical and thermal conductivity of the tissue (e.g., charring). Rather, the impedance event may be due to suboptimal contact between the electrode 22, 23 and the lesion at the electrode-tissue interface. Therefore, reducing the temperature set point during the ramp period 60 may be undesirable, as the tissue remains likely to accommodate the RF energy at a level to achieve the initial temperature set point. In other words, reducing the temperature set point during the ramp period 60 may be premature.

The electrosurgical console 12 of the present disclosure advantageously provides for limiting instances in which the temperature set point is reduced to occurrences in which the impedance events occurs after the ramp period 60. More particularly, the processor 36 is configured to determine whether the occurrence of the impedance event is during or after the ramp period 60. To do so, the processor 36 may compare the tissue temperature to the temperature set point 52 or another temperature based on the temperature set point. The temperature may be the threshold temperature previously discussed in relation to the elapsed time-based characteristic. In certain implementations, the threshold temperature may be a percentage of the temperature set point. For example, if the tissue temperature has yet to reach 85, 90, or 95% of the temperature set point 52, the processor 36 determines that the ablation procedure is in the ramp period 60. Conversely, if the tissue temperature has reached the threshold temperature or the temperature set point 52, the processor 36 determines that the ablation procedure is no longer in the ramp period 60.

The processor 36 is configured to maintain the temperature set point 52 if the occurrence of the impedance event is during the ramp period 60. With continued reference to FIG. 4, an occurrence of a ramp impedance event during the ramp period 60 is represented at 62. For the ramp impedance event 62, the processor 36 maintains the temperature set point 52 as the initial or first temperature set point ($T_1$). The processor 36 is further configured to reduce the temperature set point 52 if the occurrence of the impedance event is after the ramp period 60. An example of such an instance is the first impedance event ($I_1$) and the reduction from the first temperature set point ($T_1$) to the second temperature set point ($T_2$), as shown in FIG. 4 and as previously explained.

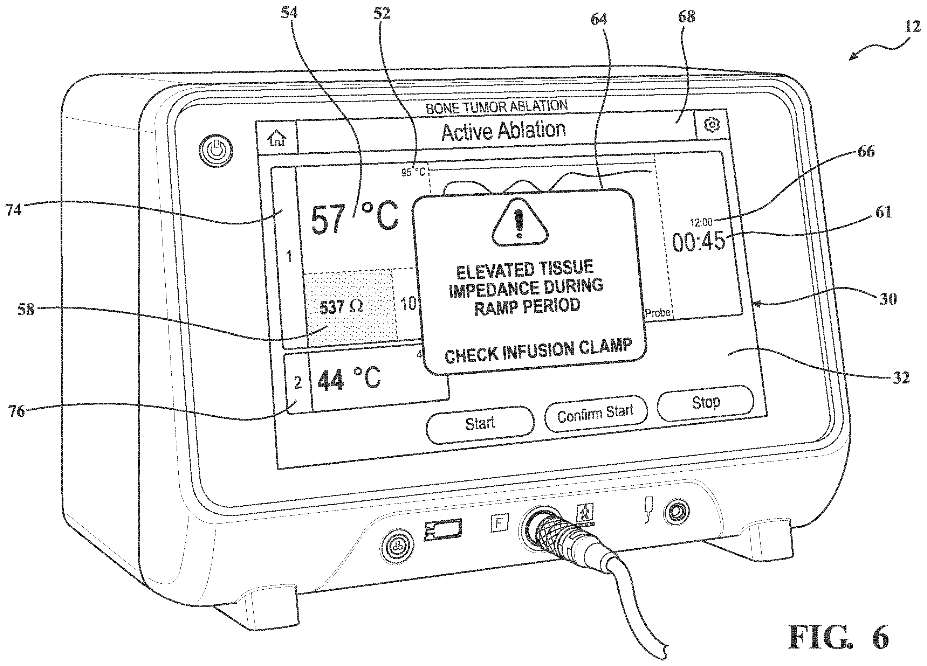

As mentioned, the impedance event during the ramp period 60 may be due to suboptimal contact between the electrode 22, 23 and the lesion at the electrode-tissue interface. The electrosurgical system 10 of the present disclosure includes the infusion module 20 to direct a fluid through the electrosurgical instrument 16 to be discharged near proximal and distal electrodes 22, 23. The discharged fluid provides improved conductivity of the RF energy across the electrode-tissue interface, which in turn limits suboptimal contact between the electrode 22, 23 and the lesion. As such, occurrences of impedance events during the ramp period 60 may be due to the user not activating the infusion module 20 or not actuating the infusion clamp 26 to permit the flow of the fluid. Therefore, in instances where the occurrence of the impedance event is during the ramp period 60, the processor 36 is further configured to instruct the display 30 to provide an alert 64. One exemplary alert 64 is shown in FIG. 6 as a pop-up notification. Additional visual alerts are contemplated as well as audible and tactile alerts. For example, FIG. 6 further shows the tissue impedance 58 being visually emphasized on the display 30 and indicative of the tissue impedance 58 exceeding the impedance threshold value for a given elapsed time-based characteristic 61 of the ablation procedure. The display 30 may also display the total ablation time 66, operating mode 68, a graphical representation 70 of a temperature versus time for the ablation procedure, an indication 72 of the electrosurgical instrument 16, 17 coupled to the electrosurgical console 12, among other treatment parameters. It is further contemplated that more than one electrosurgical instrument 16, 17 may be simultaneously operable with the electrosurgical console 12, and the display 30 may include fields 74, 76 representing each of the electrosurgical instruments 16, 17 coupled to the electrosurgical console 12 (see FIG. 1). Selection of the fields 74, 76 on the GUI 32 may permit the user to toggle between the operating parameters of each of the electrosurgical instruments 16, 17.

In certain implementations, the processor 36 may generate a secondary impedance threshold values (not identified) to be utilized in conjunction with the impedance threshold values 48, hereinafter referred to as a primary impedance threshold values. The secondary impedance threshold values may be greater than those of the primary impedance threshold values for all elapsed time-based characteristics. The secondary impedance threshold values may be utilized in instances where the reduction in the temperature set point 52 is insufficient to prevent a continued increase in tissue impedance. In other words, the secondary impedance threshold values may be considered a backup failsafe. The secondary impedance threshold values may be a function such as a curve having the same or different slope as the primary impedance threshold curve. In one example of a different slope, the secondary impedance threshold curve is a horizontal line at a constant maximum of permissible tissue impedance. Should the tissue impedance exceed the impedance threshold values of the secondary impedance threshold values, the processor 36 may instruct the controller 34 to suspend the delivery of the RF energy to the electrosurgical instrument 16, 17. The pause of the delivery of RF energy provides time for the fluid from the infusion module to partially reverse the effect of the charring of the tissue or the like. After a predefined or determined period of time, the controller 34 is configured to resume the delivery of the RF energy to the electrosurgical instrument 16, 17.

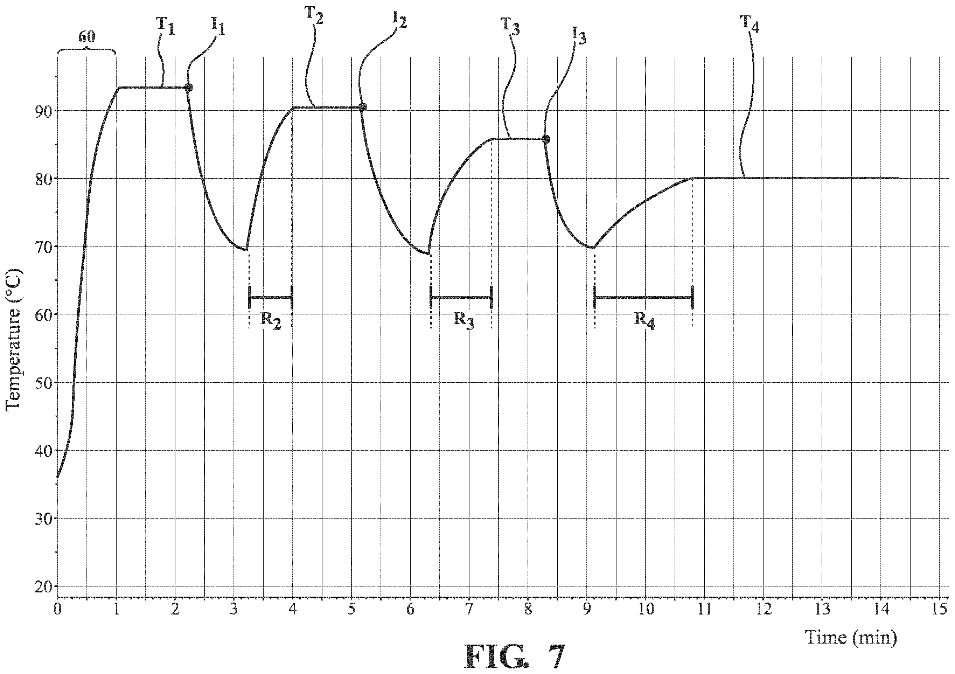

Referring now to FIG. 7, another implementation is illustrated in which the instructions 40 are executed by the processor 36 to provide for an increase in a duration of ramp periods following occurrences of impedance events. More particularly, following a temporary suspension of the delivery of the RF energy in response to an impedance event, the speed at which the tissue temperature is driven to the temperature set point is decreased. In other words, the tissue is heated more slowly following restarts at later points in the ablation procedure, as a slower ramp period provides for a more homogeneous ablation volume (i.e., less charring). Therefore, a faster ramp period may be utilized earlier in the procedure; and should charring become progressively larger problem during the ablation procedure, a less aggressive ramp may be utilized.

FIG. 7 shows the tissue temperature is ramped to the initial or first temperature set point ($T_1$), which defines the initial ramp period 60 as previously described. In response to the first impedance event ($I_1$), the processor 36 reduces the first temperature set point ($T_1$) to the second temperature set point ($T_2$). The controller 34 restarts delivery of the RF energy at a power level configured to drive the tissue temperature to the second temperature set point ($T_2$) in a second ramp period ($R_2$) having a slope of the temperature-time curve of FIG. 7. The second impedance event ($I_2$) occurs, and the processor 36 reduces the first temperature set point ($T_1$) to the second temperature set point ($T_2$). The controller 34 restarts delivery of the RF energy at another power level configured to drive the tissue temperature to the third temperature set point ($T_3$) in a third ramp period ($R_3$). The third ramp period ($R_3$) is greater in duration than the second ramp period ($R_2$), and thus the slope of the second ramp period ($R_2$) is less than the slope of the third ramp period ($R_3$). After additional delivery of the RF energy, the third impedance event ($I_3$) occurs, and the processor 36 reduces the second temperature set point ($T_2$) to the third temperature set point ($T_3$). The controller 34 restarts delivery of the RF energy at still another power level configured to drive the tissue temperature to the fourth temperature set point ($T_4$) in a fourth ramp period ($R_4$). The fourth ramp period ($R_4$) is greater in duration than the third ramp period ($R_3$), and thus the slope of the third ramp period ($R_3$) is less than the slope of the fourth ramp period ($R_4$). The remainder of the ablation procedure proceeds with the lesion being ablated at the fourth temperature set point ($T_4$).

The foregoing disclosure is not intended to be exhaustive or limit the invention to any particular form. The terminology which has been used is intended to be in the nature of words of description rather than of limitation. Many modifications and variations are possible in light of the above teachings and the invention may be practiced otherwise than as specifically described. It should be understood that treatment parameters other than impedance and temperature may be utilized with the aforementioned techniques. Furthermore, it is contemplated that the treatment parameters may be controlled or defined in manners different from the techniques described. It is further understood that the objects of the present disclosure may be used with microwave energy or electrical energy other than radiofrequency energy.

Exemplary methods of performing an electrosurgical procedure with the electrosurgical system 10 of the present disclosure is described with reference to the following clauses.

Clause 1 - A method of operating an electrosurgical console for ablating tissue during an ablation procedure with an electrosurgical instrument, the method including: controlling the electrical energy delivered from the electrode based on a temperature measurement of the thermocouple and a temperature set point at which the tissue is to be ablated; determining an initial tissue impedance value; determining impedance threshold values based on the initial tissue impedance value, wherein the impedance threshold values vary based on an elapsed time-based characteristic of the ablation procedure; determining subsequent tissue impedance values during delivery of the electrical energy with the electrosurgical instrument; determining an occurrence of an impedance event by comparing the subsequent tissue impedance values with the threshold values; and reducing the temperature set point based on the occurrence of the impedance event.

Clause 2 - The method of clause 1, further including determining the elapsed time-based characteristic by determining a percentage of total procedure time of which the tissue is above a threshold temperature.

Clause 3 - The method of clause 1 or 2, wherein the ablation procedure includes a temperature set point at which the tissue is to be ablated, and a ramp period during which the tissue temperature is increasing towards the temperature set point, the method further including determining whether the occurrence of the impedance event is during or after the ramp period, and maintaining the temperature set point if the occurrence of the impedance event is during the ramp period.

Clause 4 - The method of clause 3, further including reducing the temperature set point if the occurrence of the impedance event is after the ramp period.

Clause 5 - The method of clause 4, further including reducing the temperature set point by a fixed temperature offset with the occurrence of the impedance event and subsequent occurrences of additional impedance events.

Clause 6 - The method of any one of clauses 3-5, further including displaying an alert if the occurrence of the impedance event is during the ramp period.

Clause 7 - The method of any one of clauses 3-5, further including suspending delivery of the electrical energy based on the occurrence of the impedance event after the ramp period, and restarting the delivery of the electrical energy after a fixed period.

Clause 8 - The method of clause 7, further including, upon the restart, delivering the electrical energy with power to re-ramp the tissue temperature to the reduced temperature set point, and, optionally, wherein the re-ramp is longer at later points in the ablation procedure.

Clause 9 - The method of any one of clauses 1-8, further including determining a secondary impedance threshold values, and suspending delivery of the electrical energy based on the subsequent tissue impedance values exceeding secondary threshold values.

Clause 10 - A method of operating an electrosurgical console for ablating tissue during an ablation procedure with an electrosurgical instrument in which the ablation procedure includes a temperature set point at which the tissue is to be ablated, and a ramp period during which tissue temperature is increasing towards the temperature set point, the method including: determining impedance threshold values; determining tissue impedance values during delivery of the electrical energy with the electrosurgical instrument; determining an occurrence of an impedance event by comparing the tissue impedance values with the impedance threshold values; determining whether the occurrence of the impedance event is during or after the ramp period; and maintaining the temperature set point if the occurrence of the impedance event is during the ramp period.

Clause 11 - The method of clause 10, further including reducing the temperature set point if the occurrence of the impedance event is after the ramp period.

Clause 12 - The method of clause 11, further including reducing the temperature set point by a fixed temperature offset with the occurrence of the impedance event and subsequent occurrences of additional impedance events.

Clause 13 - The method of any one of clauses 9-12 further including receiving tissue temperature values, and determining whether the occurrence of the impedance event is during or after the ramp period by comparing the tissue temperature values with the temperature set point or a temperature threshold based on the temperature set point.

Clause 14 - The method of any one of clauses 9-13, further including controlling the delivery of the electrical energy from the electrosurgical instrument based on the occurrence of the impedance event.

Clause 15 - The method of any one of clauses 9-14, further including displaying an alert if the occurrence of the impedance event is during the ramp period.

Clause 16 - The method of any one of clauses 9-15, further including suspending delivery of the electrical energy based on the occurrence of the impedance event after the ramp period, and restarting the delivery of the electrical energy after a fixed period.

Clause 17 - The method of clause 16, further, upon the restart, delivering the electrical energy with power to re-ramp the tissue temperature to the reduced temperature set point, and, optionally, wherein the re-ramp is longer at later points in the ablation procedure.

Clause 18 - A method of operating an electrosurgical console for ablating tissue during an ablation procedure with an electrosurgical instrument in which the ablation procedure includes a temperature set point at which the tissue is to be ablated, and a ramp period during which tissue temperature is increasing towards the temperature set point, the method including: determining tissue impedance values during delivery of the electrical energy with the electrosurgical instrument; determining an occurrence of an impedance event by comparing the tissue impedance values with an impedance threshold; and reducing the temperature set point by a fixed temperature offset after the occurrence of the impedance event and subsequent occurrences of the impedance event.

Clause 19 - The method of clause 18, further including determining whether the occurrence of the impedance event is during or after the ramp period, and reducing the temperature set point if the occurrence of the impedance event is after the ramp period.

Clause 20 - The method of clause 19, further including suspending the electrical energy being delivered to the tissue, and optionally, upon a restart, to delivering the electrical energy with power to re-ramp the tissue temperature to the reduced temperature set point with the re-ramp being longer at later points in the ablation procedure.

The following examples are also encompassed by the present disclosure and may fully or partly be incorporated into embodiments.

1. An electrosurgical console for ablating tissue during an ablation procedure with an electrosurgical instrument that includes an electrode for delivering electrical energy to the tissue, and a thermocouple, the electrosurgical console comprising:

    a console configured to be coupled with the electrosurgical instrument and comprising a controller configured to be in communication with the electrosurgical instrument with the electrosurgical instrument coupled to the console; and
a processor configured to be in communication with the controller, wherein the processor is further configured to:

    cause the controller to control the electrical energy delivered from the electrode based on a tissue temperature as sensed by the thermocouple and a temperature set point at which the tissue is to be ablated;
determine an initial tissue impedance value;
determine impedance threshold values based on the initial tissue impedance value, wherein the impedance threshold values = vary based on an elapsed time-based characteristic of the ablation procedure;
determine subsequent tissue impedance values during delivery of the electrical energy with the electrosurgical instrument;
determine an occurrence of an impedance event by comparing the subsequent tissue impedance values with the impedance threshold values; and
reduce the temperature set point based on the occurrence of the impedance event.

2. The electrosurgical console of claim 1, wherein the initial tissue impedance value is based on at least one of an electrical parameter associated with the electrode, and the tissue temperature.

3. The electrosurgical console of claim 1, wherein the impedance threshold values define an impedance threshold curve that is linear with the impedance threshold values being greater with higher values of the elapsed time-based characteristic.

4. The electrosurgical console of claim 3, wherein the impedance threshold curve has a slope based on the initial tissue impedance value and a final impedance threshold value with the slope being different than another impedance threshold curve having a different initial threshold value.

5. The electrosurgical console of any one of claims 1-4, wherein the processor is further configured to determine the elapsed time-based characteristic by determining a percentage of total procedure time of which the tissue is above a threshold temperature.

6. The electrosurgical console of claim 5, wherein the threshold temperature is a temperature set point or another temperature based on the temperature set point.

7. The electrosurgical console of any one of claims 1-6, wherein the ablation procedure includes a temperature set point at which the tissue is to be ablated, and a ramp period during which the tissue temperature is increasing towards the temperature set point, wherein the processor is configured to determine whether the occurrence of the impedance event is during or after the ramp period, maintain the temperature set point if the occurrence of the impedance event is during the ramp period.

8. The electrosurgical console of claim 7, wherein the processor is further configured to reduce the temperature set point if the occurrence of the impedance event is after the ramp period.

9. The electrosurgical console of claim 8, wherein the processor is further configured to reduce the temperature set point by a fixed temperature offset with the occurrence of the impedance event and subsequent occurrences of additional impedance events.

10. The electrosurgical console of claim 9, wherein the fixed temperature offset is within a range of three to seven degrees Celsius.

11. The electrosurgical console of any one of claims 7-11, further comprising a display in communication with the processor, wherein the processor is further configured to instruct the display to provide an alert if the occurrence of the impedance event is during the ramp period.

12. The electrosurgical console of any one of claims 7-11, wherein the processor is further configured to instruct the controller to suspend delivery of the electrical energy based on the occurrence of the impedance event after the ramp period, and restart the delivery of the electrical energy after a fixed period.

13. The electrosurgical console of claim 12, wherein the processor is further configured to instruct the controller, upon the restart, to deliver the electrical energy with power to re-ramp the tissue temperature to the reduced temperature set point.

14. The electrosurgical console of claim 13, wherein the re-ramp is longer at later points in the ablation procedure.

15. The electrosurgical console of any one of claims 1-11, wherein the processor is further configured to determine secondary impedance threshold values greater than the impedance threshold values for each of the elapsed time-based characteristic, wherein the processor is further configured to instruct the controller to suspend delivery of the electrical energy based on the subsequent tissue impedance values exceeding the secondary impedance threshold values.

16. An electrosurgical console for an ablation procedure with an electrosurgical instrument that includes an electrode for delivering electrical energy to tissue, a thermocouple, wherein the ablation procedure includes a temperature set point at which the tissue is to be ablated, and a ramp period during which tissue temperature is increasing towards the temperature set point, the electrosurgical console comprising:

a console configured to be coupled with the electrosurgical instrument and comprising a controller configured to be in communication with the electrosurgical instrument with the electrosurgical instrument coupled to the console; and
a processor configured to be in communication with the controller, wherein the processor is further configured to:

determine impedance threshold values;
determine tissue impedance values during delivery of the electrical energy with the electrosurgical instrument;
determine an occurrence of an impedance event by comparing the tissue impedance values with the impedance threshold values;
determine whether the occurrence of the impedance event is during or after the ramp period; and
maintain the temperature set point if the occurrence of the impedance event is during the ramp period.

17. The electrosurgical console of claim 16, wherein the processor is further configured to reduce the temperature set point if the occurrence of the impedance event is after the ramp period.

18. The electrosurgical console of claim 17, wherein the processor is further configured to reduce the temperature set point by a fixed temperature offset with the occurrence of the impedance event and subsequent occurrences of additional impedance events.

19. The electrosurgical console of any one of claims 16-18, wherein the fixed temperature offset is within a range of three to seven degrees Celsius.

20. The electrosurgical console of any one of claims 16-19, wherein the processor is further configured to:

receive tissue temperature values from the thermocouple; and

determine whether the occurrence of the impedance event is during or after the ramp period by comparing the tissue temperature values with the temperature set point or a temperature threshold based on the temperature set point.

21. The electrosurgical console of any one of claims 16-20, wherein the controller is further configured to control the delivery of the electrical energy from the electrosurgical instrument based on the occurrence of the impedance event.

22. The electrosurgical console of any one of claims 16-21, further comprising a display in communication with the processor, wherein the processor is further configured to instruct the display to provide an alert if the occurrence of the impedance event is during the ramp period.

23. The electrosurgical console of any one of claims 16-22, wherein the processor is further configured to instruct the controller to suspend delivery of the electrical energy based on the occurrence of the impedance event after the ramp period, and restart the delivery of the electrical energy after a fixed period.

24. The electrosurgical console of claim 23, wherein the processor is further configured to instruct the controller, upon the restart, to deliver the electrical energy with power to re-ramp the tissue temperature to the temperature set point.

25. The electrosurgical console of claim 24, wherein the re-ramp is longer at later points in the ablation procedure.

26. An electrosurgical console for an ablation procedure with an electrosurgical instrument that includes an electrode for delivering electrical energy to tissue, a thermocouple, wherein the ablation procedure includes a temperature set point at which the tissue is to be ablated, and a ramp period during which tissue temperature is increasing towards the temperature set point the electrosurgical console comprising:

a console configured to be coupled with the electrosurgical instrument; and
a processor configured be in operable communication with the electrosurgical instrument with the electrosurgical instrument coupled to the console, wherein the processor is further processor to:

determine tissue impedance values during delivery of the electrical energy with the electrosurgical instrument;

determine an occurrence of an impedance event by comparing the tissue impedance values with an impedance threshold; and
reduce the temperature set point by a fixed temperature offset after the occurrence of the impedance event and subsequent occurrences of the impedance event.

27. The electrosurgical console of claim 26, wherein the processor is further configured to:

determine whether the occurrence of the impedance event is during or after the ramp period; and
reduce the temperature set point if the occurrence of the impedance event is after the ramp period.

28. The electrosurgical console of claim 26 or 27, wherein the fixed temperature offset is within a range of three to seven degrees Celsius.

29. The electrosurgical console of claim 28, wherein the fixed temperature offset is five degrees Celsius.

30. The electrosurgical console of any one of claims 26-29, wherein the console comprises a controller configured to be in communication with the processor and in communication with the electrosurgical instrument with the electrosurgical instrument coupled to the console, wherein the controller is configured to control the delivery of the electrical energy from the electrosurgical instrument based on the occurrence of the impedance event.

31. The electrosurgical console of claim 30, wherein the processor is further configured to instruct the controller to reduce or suspend the electrical energy being delivered to the tissue.

32. The electrosurgical console of claim 31, wherein the processor is further configured to instruct the controller, upon a restart, to deliver the electrical energy with power to re-ramp the tissue temperature to the reduced temperature set point.

33. The electrosurgical console of claim 32, wherein the re-ramp is longer at later points in the ablation procedure.

34. The electrosurgical console of any one of claims 26-33, wherein the reduced temperature set point is configured to be constant for a remainder of the ablation procedure in absence of the subsequent occurrences of the impedance event.

**Claims**

1. An electrosurgical console (12) for an ablation procedure with an electrosurgical instrument (16, 17) that includes an electrode (22, 23) for delivering electrical energy to tissue, a thermocouple (24), wherein the ablation procedure includes a temperature set point (52) at which the tissue is to be ablated, and a ramp period (60, $R_2$, $R_3$, $R_4$) during which tissue temperature is increasing towards the temperature set point, the electrosurgical console comprising:

   a console configured to be coupled with the electrosurgical instrument and comprising a controller (34) configured to be in communication with the electrosurgical instrument with the electrosurgical instrument coupled to the console; and
   a processor (36) configured to be in communication with the controller, wherein the processor is further configured to:

   determine impedance threshold values (44, 48, 50);
   determine tissue impedance values (56, 58) during delivery of the electrical energy with the electrosurgical instrument;
   determine an occurrence of an impedance event ($I_1$, $I_2$, $I_3$, $I_N$) by comparing the tissue impedance values with the impedance threshold values,

   **characterized in that** the processor is further configured to:

   determine whether the occurrence of the impedance event is during or after the ramp period; and
   maintain the temperature set point if the occurrence of the impedance event is during the ramp period.

2. The electrosurgical console of claim 1, wherein the processor is further configured to reduce the temperature set point if the occurrence of the impedance event is after the ramp period.

3. The electrosurgical console of claim 2, wherein the processor is further configured to reduce the temperature set point by a fixed temperature offset with the occurrence of the impedance event and subsequent occurrences of additional impedance events.

4. The electrosurgical console of any one of claims 1-3, wherein the fixed temperature offset is within a range of three to seven degrees Celsius, and optionally five degrees Celsius.

5. The electrosurgical console of any one of claims 1-4, wherein the processor is further configured to:

   receive tissue temperature values from the thermocouple; and
   determine whether the occurrence of the impedance event is during or after the ramp period by comparing the tissue temperature values with the temperature set point or a temperature threshold based on the temperature set point.

6. The electrosurgical console of any one of claims 1-5, wherein the controller is further configured to control the delivery of the electrical energy from the electrosurgical instrument based on the occurrence of the impedance event.

7. The electrosurgical console of any one of claims 1-6, further comprising a display (30) in communication with the processor, wherein the processor is further configured to instruct the display to provide an alert (64) if the occurrence of the impedance event is during the ramp period.

8. The electrosurgical console of claim 7, further comprising an infusion module (20) comprising an infusion clamp (26) configured to be actuated to selectively start and stop the flow of the fluid through the electrosurgical instrument, wherein the alert comprises a pop-up notification to check the infusion clamp.

9. The electrosurgical console of any one of claims 1-7, wherein the processor is further configured to instruct the controller to suspend delivery of the electrical energy based on the occurrence of the impedance event after the ramp period, and restart the delivery of the electrical energy after a fixed period.

10. The electrosurgical console of claim 9, wherein the processor is further configured to instruct the controller, upon the restart, to deliver the electrical energy with power to re-ramp the tissue temperature to the temperature set point.

11. The electrosurgical console of claim 10, wherein the re-ramp is longer at later points in the ablation procedure.

12. The electrosurgical console of claim 3, wherein the reduced temperature set point is configured to be constant for a remainder of the ablation procedure in absence of the subsequent occurrences of the impedance event.

13. The electrosurgical console of any one of claims 1-12, wherein an initial one of the tissue impedance values is based on at least one of an electrical parameter associated with the electrode, and the tissue temperature.

14. The electrosurgical console of any one of claims 1-13, wherein the impedance threshold values define an impedance threshold curve (46) that is linear with the impedance threshold values being greater with higher values of the elapsed time-based characteristic.

15. The electrosurgical console of claim 14, wherein the impedance threshold curve has a slope based on the initial tissue impedance value (44) and a final impedance threshold value (50) with the slope being different than another impedance threshold curve having a different initial threshold value.

**FIG. 1**

FIG. 2

FIG. 3

FIG. 4

**FIG. 5**

BONE TUMOR ABLATION

Active Ablation

57 °C

95 °C

537 Ω

10

44 °C

12:00

00:45

Probe

ELEVATED TISSUE
IMPEDANCE DURING
RAMP PERIOD

CHECK INFUSION CLAMP

Start

Confirm Start

Stop

F

FIG. 6

**FIG. 7**

FIG. 8

EP 4 631 450 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63227387
- WO 2020198150 A
- WO 2018200254 A